# EUROPÄISCHE PATENTSCHRIFT

(11) **EP 1 294 908 B1**
(45) Veröffentlichungstag und Bekanntmachung des Hinweises auf die Patenterteilung: **08.03.2006**
(21) Anmeldenummer: 01955217.3
(22) Anmeldetag: 28.06.2001
(51) Int. Cl.: C12N 15/67

(54) **VERFAHREN ZUR VERBESSERUNG DER TRANSFEKTIONSEFFIZIENZ**
METHOD FOR IMPROVING TRANSFECTION EFFICIENCY
PROCEDE PERMETTANT D'AMELIORER LE RENDEMENT DE TRANSFECTION

(30) Priorität: 28.06.2000 DE 10031900; 18.08.2000 DE 10040895
(43) Veröffentlichungstag der Anmeldung: 26.03.2003
(73) Patentinhaber: MAX-DELBRÜCK-CENTRUM FÜR MOLEKULARE MEDIZIN, 13125 Berlin (DE)
(72) Erfinder: CARTIER, Regis, 13125 Berlin (DE); BÖTTGER, Michael, 13189 Berlin (DE); HABERLAND, Annekathrin, 10430 Berlin (DE); RESZKA, Regina, 16341 Schwanebeck (DE)
(74) Vertreter: Hertin, Paul W.
(86) Internationale Anmeldenummer: PCT/DE2001/002336
(87) Internationale Veröffentlichungsnummer: WO 2002/000991

(56) Entgegenhaltungen:
- EP-A- 0 545 016
- WO-A-94/23751
- WO-A-98/35984
- WO-A-99/53961
- US-A- 5 736 392
- US-A- 6 022 735

## Beschreibung

Die Erfindung betrifft ein Verfahren zur Verbesserung der Transfektionseffizienz unter Verwendung von K16-Peptiden. Desweiteren betrifft die Erfindung neue K16-Peptide. Anwendungsgebiete sind die Molekularbiologie und die Medizin.

Der Transfektionsmechanismus nicht-viraler Gentransfer-Systeme ist ein komplexer Prozess, der durch eine Vielzahl biologischer Barrieren gehemmt wird. Ein wesentlicher limitierender Schritt ist die Überwindung der Zellkernmembran durch den Vektor. Durch diesen Schritt gelangt die transgene DNA in den Zellkern, wodurch erst eine effiziente Expression des Transgens möglich wird.
Um den Transfer von Plasmid-DNA in den Zellkern zu verbessern, wird u. a. versucht, Kerntransportmechanismen der Zelle zu nutzen Eine Strategie besteht darin, peptidische NLS-Sequenzen nicht-kovaIent an die DNA über eine zusätzlich angefügte kationische DNA-Bindungssequenz zu koppeln, mit dem Ziel, daß die Kerntransport-Maschinerie der Zelle diese an DNA gebundene NLS-Sequenzen erkennt und zusammen mit der DNA in den Zellkern transportiert.
Die in nicht-viralen Gentransfersystemen meist verwendete NLS-Sequenz ist der Aminosäurenabschnitt 126 bis 132 des Simian Virus 40 (SV 40) large T-Antigens (SV40-NLS). Eine Sequenz-spezifische Erhöhung der Transfektionseffizienz durch diese NLS-Sequenz konnte bisher in der oben erwähnten Strategie nicht nachgewiesen werden.

WO 99/53961, WO 98/35984, WO 94/23751 beschreiben die Transfektion mittels polykationischer Peptide. US 6,022,735 offenbart ebenfalls Polylysinhaltige Zusammensetzungen als Transfektions agentien.

Der Erfindung liegt die Aufgabe zugrunde, den Kemtransport von Plasmid-DNA zu verbessem.Gemäß der Erfindung wird ein synthetisches Peptid bestehend aus einer oligo-Lysin-DNA-Bindungssequenz und einer SV40-NLS konstruiert Femer wird die Konstruktion und Optimierung eines Komplexes bestehend aus Plasmid-DNA, synthetischem Peptid und Lipid realisiert.
Als synthetische Peptide wurden die folgenden Peptide ausgewählt:

K16-NLS: CKKKKKKKKKKKKKKKKGGGPKKKRKVG
K16-SLN: CKKKKKKKKKKKKKKKKGGGGVKRKKKP
K16-Vpr: KKKKKKKKKKKKKKKKCGGGIGCRHSRIGVTRQRRARNGASRS
K16-VprN KKKKKKKKKKKKKKKKGGGCNEWTLELLEELKNEAVRHF
K16-mVprN KKKKKKKKKKKKKKKKGGGNEATLELLPELKNPAVRHF

Alle diese Peptide tragen 16 Lysin-Reste zur Bindung der Peptide an die Plasmid-DNA.

Das K16-NLS Peptid trägt außerdem die SV40-NLS-Sequenz (PKKKRKV). Das KI6-SLN-Peptid ist ein Kontrollpeptid mit gleicher DNA-Bindungssequenz und identischer Aminosäuren-Zusammensetzung , das eine umgedrehte SV40-NLS-Sequenz trägt. Das K16-Vpr-Peptid trägt zusätzlich zur Oligo-Lysin-DNA-Bindungssequenz eine NLS-Sequenz, die aus der C-tenninalen Region des HIV-Vpr-Protein abgeleitet wurde. Das K16-VprN-Peptid trägt analog eine Sequenz, die aus der N-tenninalen Region abgeleitet wurde. Das K16-mVprN-Peptid ist ein Kontrollpeptid mit gleicher DNA-Bindungssequenz und einer VprN-Kontrollsequenz, die durch einen Austausch von drei Aminosäuren konstruiert wurde und dadurch als defizient für den erleichterten Kernimport beschrieben wurde.

Außerdem wurde ein synthetisches Peptid bestehend ausschliesslich aus 16 Lysinresten (K16) als zusätzliches Kontrollpeptid verwendet.

Als Lipid wurde bevorzugt die Lipid-Zusammensetzung Lipofektin verwendet.

Es wurden Komplexe aus Plasmid-DNA, Peptid und Lipid hergestellt und zur Transfektion von humanen Kolon-Karzinoma-Zellen (HCT 116) getestet. Zur Messung der Transfektionseffizienz wurde das Luziferase-Reportergen eingesetzt.

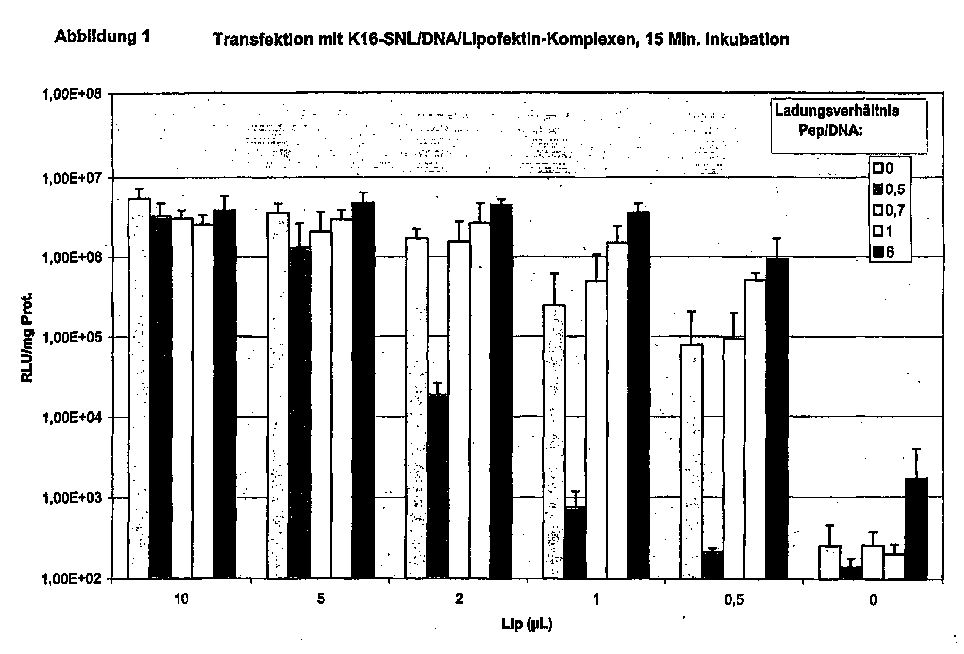

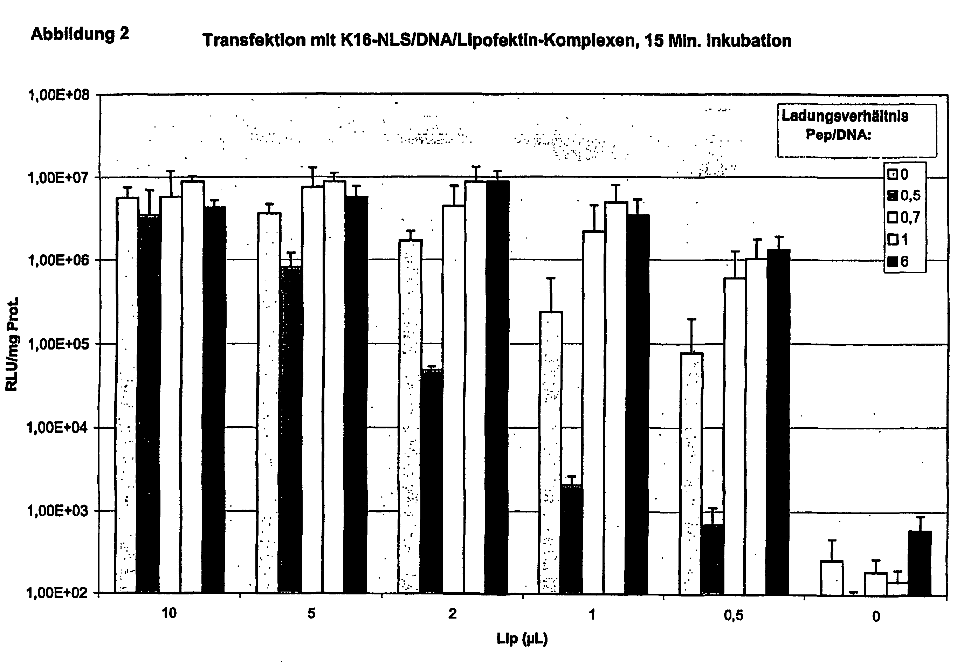

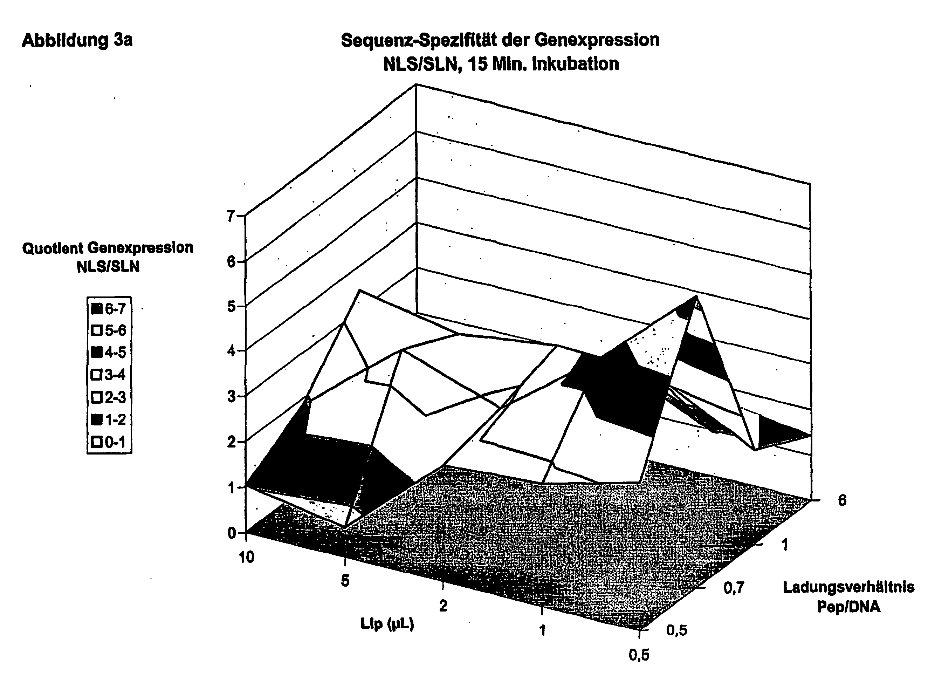

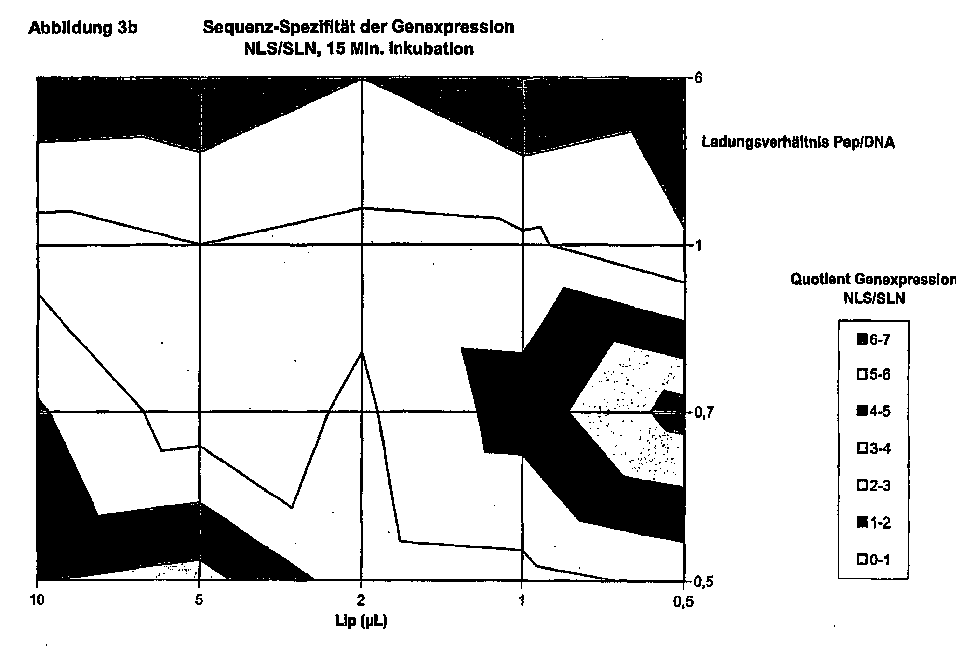

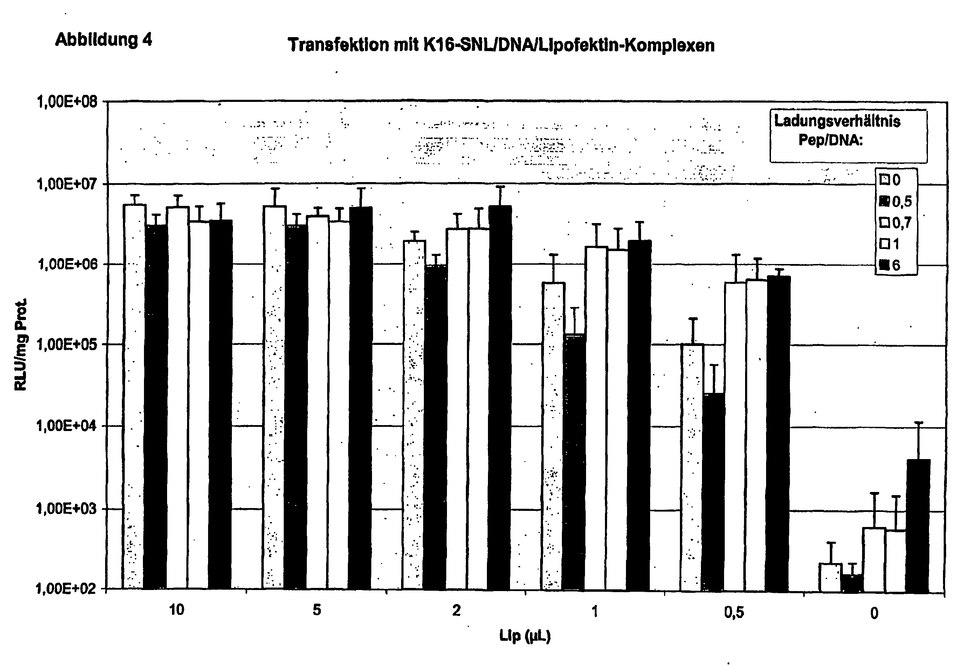

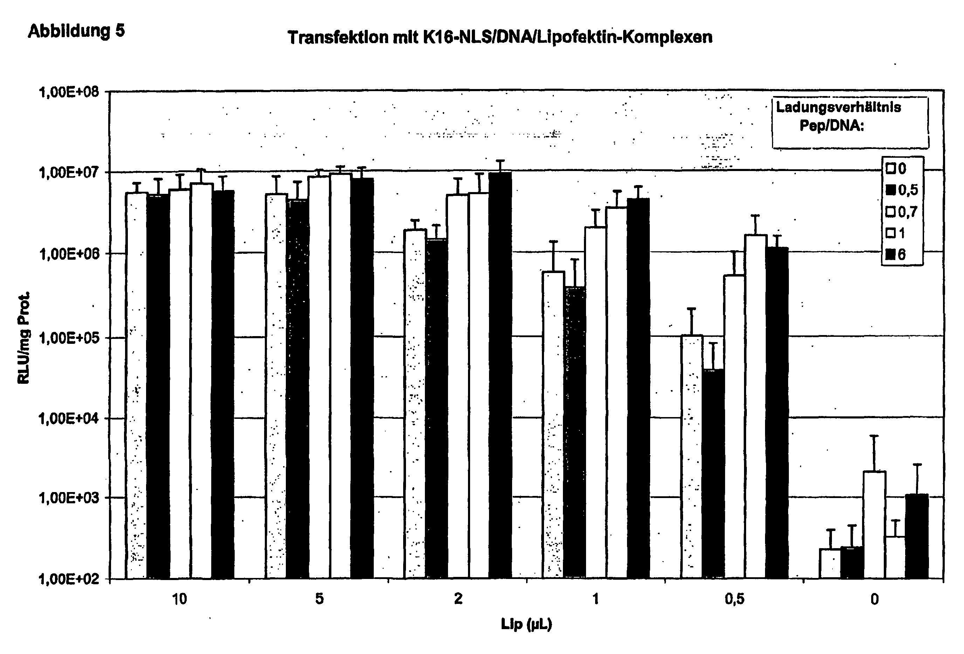

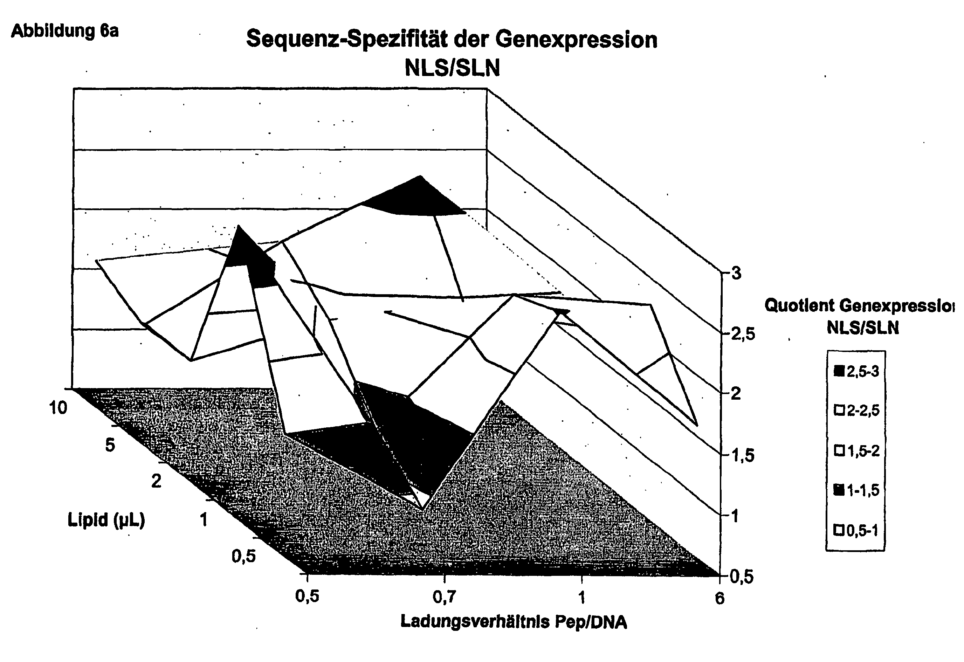

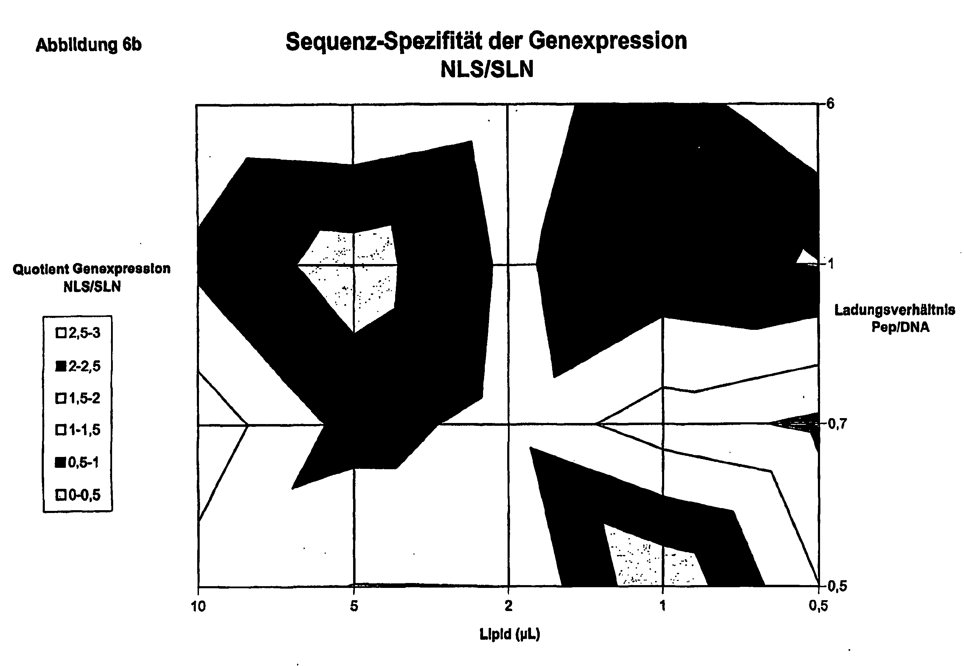

Außer der Konstruktion der K16-NLS-Peptide besteht die erfinderische Leistung in der Konstruktion des DNA-Peptid-Lipid-Komplexes. Dabei sind Zusammensetzung der Komplexe durch die Auswahl bestimmter Ladungsverhaltnisse zwischen Peptid und DNA sowie die Menge an Lipid entscheidend. Das Verfahren der Konstruktion ist ebenfalls ein entscheidender Faktor (Konzentrationen, Volumina, Puffersystem, Inkubationszeiten und Reihenfolge der Konstruktion, vgL die Abbildungen 1-3 und 4-6).
Nach Optimierung wurde eine Zusammensetzung und eine Verfahrensweise entwickelt, die als Ergebnis eine Sequenz-spezifische Erhöhung der Genexpression des Reportergens nach Transfektion von HCT 116-Zellen hatte.
Die erfinderische Leistung besteht darin, das Peptid-DNA-Verhältnis so zu wählen, das zunächst ein unstabiler Peptid-DNA-Komplex hergestellt wird, dem danach eine relativ kleine Menge an Lipid beigefügt wird.

Die generierte Sequenz-spezifische Erhöhung der Genexpression korreliert mit einem erhöhten Kemtransport der DNA.

Ferner wurde erfindungsgemäß ein zyklisches Peptid zur Stabilisierung der NLS-Sequenz und seine Verwendung zur Optimierung eines nicht-viralen Gentransfersystems konstruiert.

Die SV40-NLS-Sequenz ist durch einen hohen Anteil an basischen Resten stark positiv geladen. Wie in Transfektionsexperimenten und durch biochemische Analyse nachgewiesen wurde, interagiert sehr wahrscheinlich die SV40-NLS mit der Plasmid-DNA. Die Bindung der SV40-NLS-Sequenz an die DNA kann potentiel dazu führen, daß das Peptid einerseits für eine Interaktion mit der Nukleartransport-Machinerie der Zelle maskiert ist und andererseits seine Sekundärstruktur verändert wird, so daß es als Kernimport-Substrat nicht mehr erkannt werden könnte.

Die erfinderische Idee besteht darin, die Sekundärstruktur der SV40-NLS-Sequenz durch Zyklisierung des Peptids zu stabilisieren. Dabei wurde die Interaktion der SV40-NLS-Sequenz mit seinem Rezeptor Importin a in einer Faltblattstruktur-Konformation ausgenutzt. Als Basis der Zyklisierung diente daher ein zyklisches Peptid, welches aus zwei antiparallel liegenden Faltblattsequenzen besteht

Die Erfindung besteht darin, in diesem zyklischen Peptid die SV40-NLS-Sequenz zu rekonstruieren. Das Ergebnis ist ein zyklisches Peptid bestehend aus zwei antiparallelen Faltblatt-Strukturen, wobei die eine Seite die SV40-NLS-Sequenz enthält. Zur Bindung an die DNA wurden an der gegenüberliegenden Seite der SV40-NLS-Sequenz kovalent 16 Lysin-Reste gebunden. Das resultierende Konstrukt ist also ein synthetisches Peptid mit einer zyklisierten NLS-Sequenz und einer oligo-Lysin-DNA-Bindungsstelle.

Es hat die folgende

Peptid-Sequenz:
K16-CYC: - VKLKVYPLKKKRKP -
KKKKKKKKKKKKKKKKC

Gegenstand der Erfindung sind desweiteren zwei neue K16-Peptide, das insgesamt 39 Aminosäuren aufweisende Peptid mit der Sequenz: K16-VprN KKKKKKKKKKKKKKKKGGGCNEWTLELLEELKNEAVRHF und das insgesamt 38 Aminosäuren aufweisende Peptid mit der Sequenz:

K16-mVprN KKKKKKKKKKKKKKKKGGGNEATLELLPELKNPAVRHF.

Diese Peptide sind sowohl in Kombination mit Plasmid-DNA als auch in Kombination mit Plasmid-DNA und Lipid zur Transfektion geeignet

Die Erfindung soll nachfolgend durch Ausfühmngsbeispiele näher erläutert werden.

### Verwendung des K16-CYC-Peptids in einem nicht-viralen Gentransfer-System:

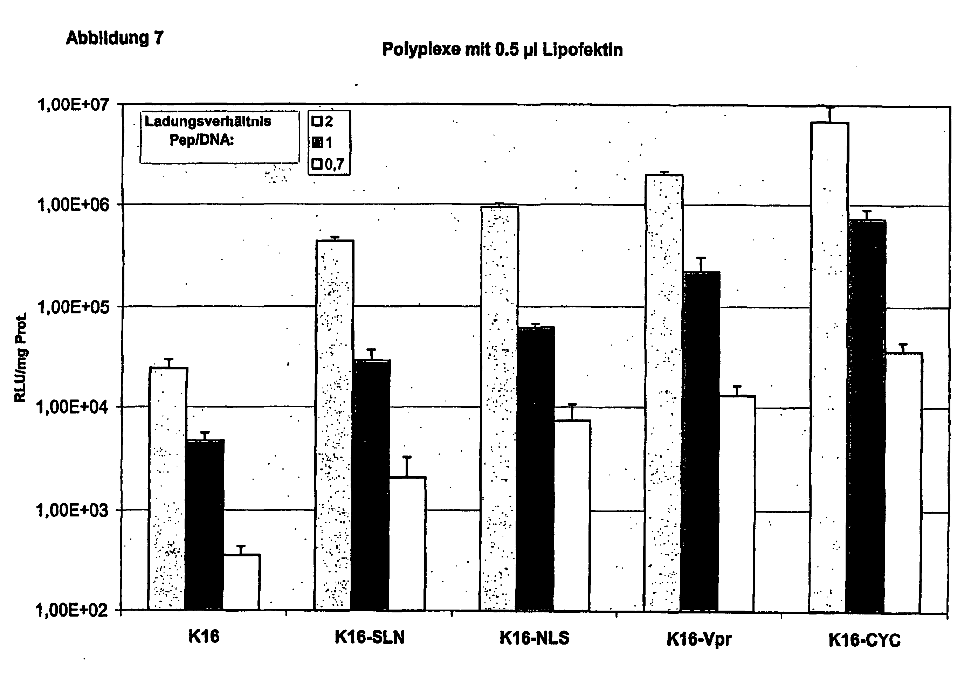

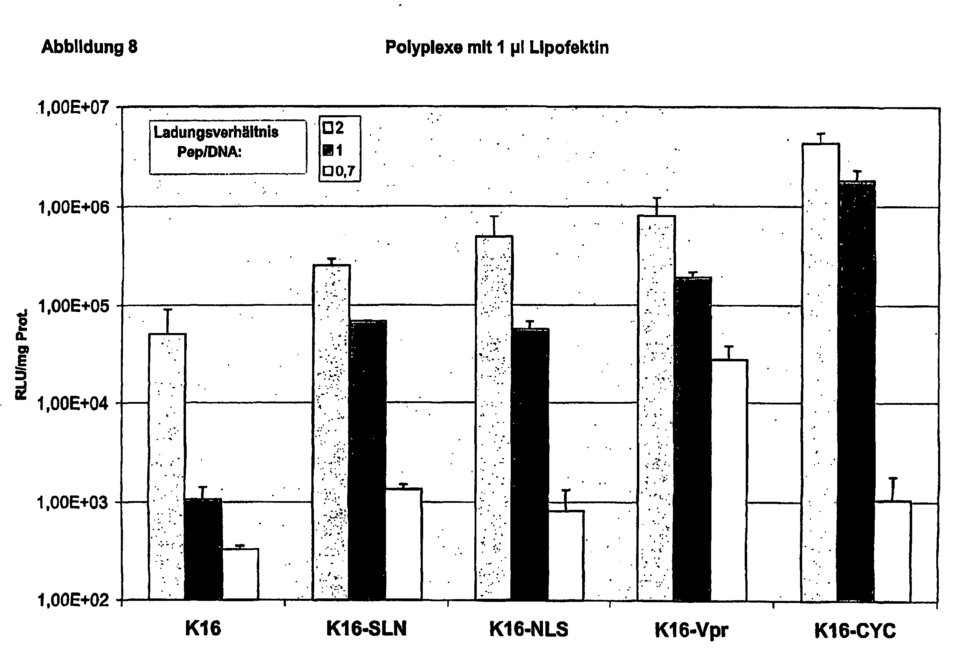

Auf der Grundlage der oben beschriebenen Optimierung eines DNA-Peptid-Lipid Komplexes wurden Komplexe bestehend aus Plasmid-DNA, dem K16-CYC-Peptid und Lipofektin hergestellt und zur Transfektion von HCT116-Zellen getestet.
Das Ergebnis zeigt eine drastische Erhöhung der Genexpression nach Transfektion mit dem Kl 6-CYC-Peptid im Vergleich zum K16-NLS-Peptid, das eine lineare Form der SV40-Sequenz trägt(vgl. Abbildungen 7 und 8).

### Verwendung des K16-Vprn N-Peptids in einem nicht-viralen Gentransfer-System;

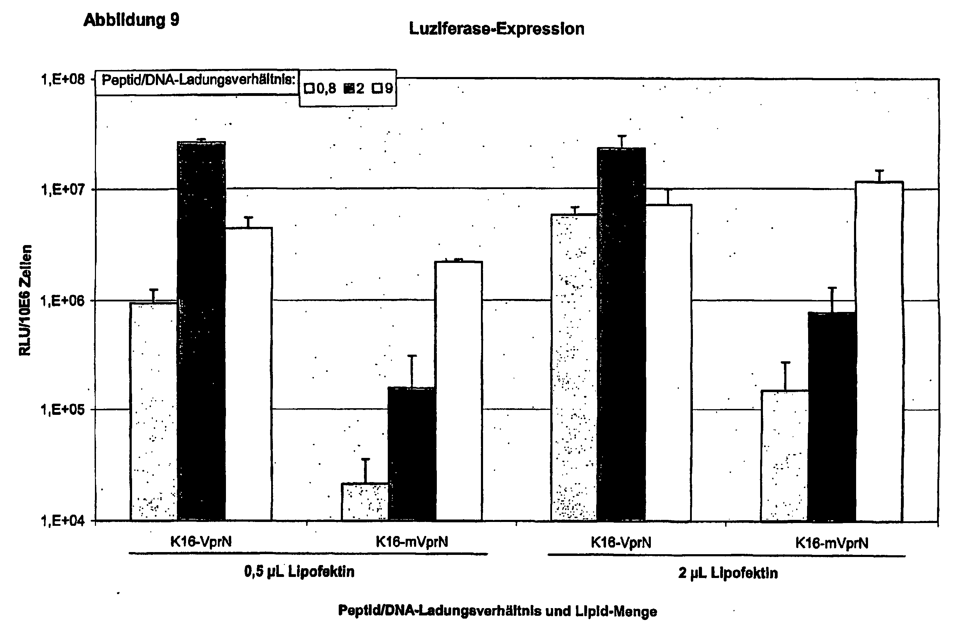

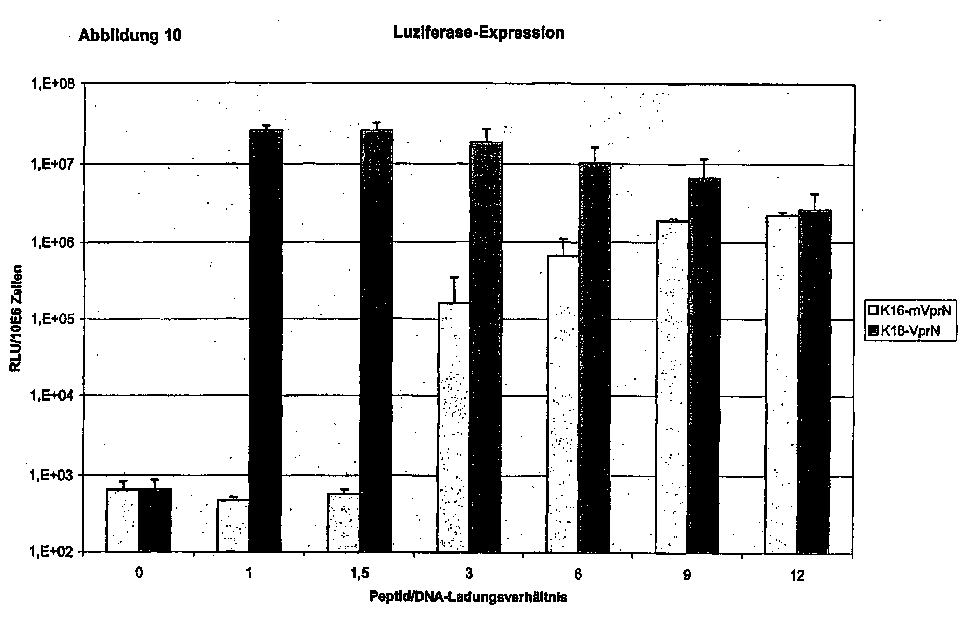

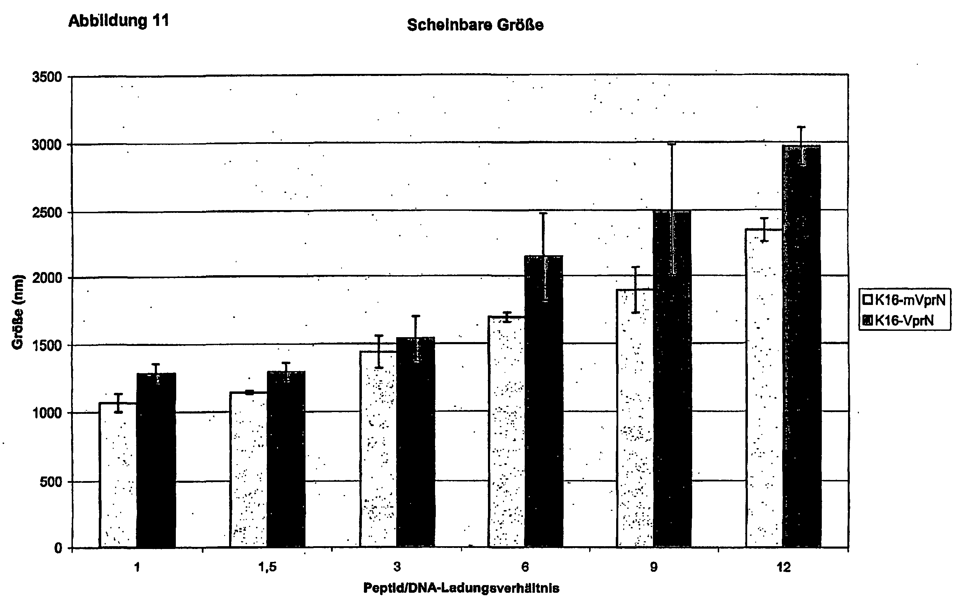

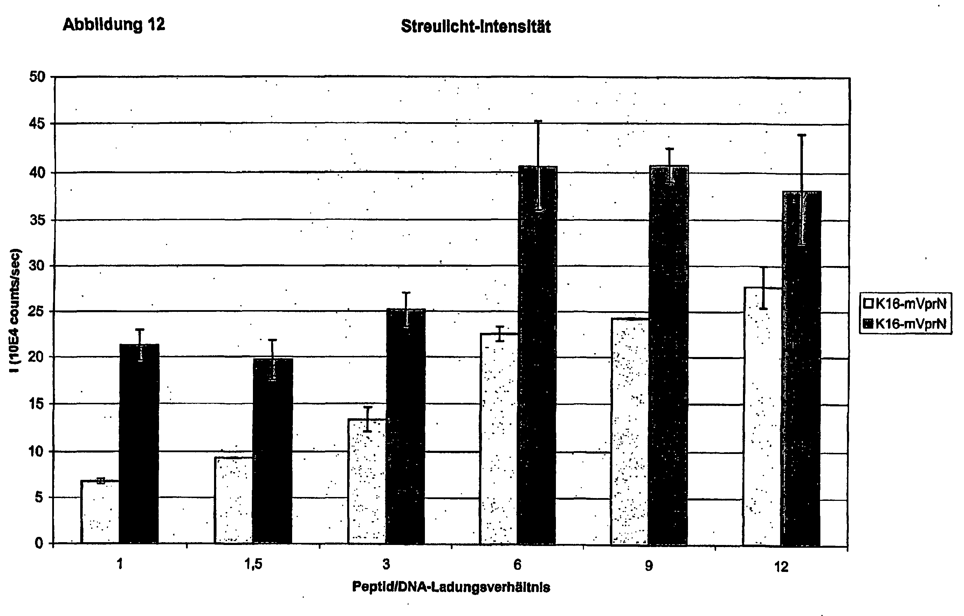

Es wurden Komplexe bestehend aus Plasmid-DNA, dem K16-VprN-Peptid und Lipofektin hergestellt und zur Transfektion von HCT116-Zellen getestet.
In einer weiteren Anwendung wurden Komplexe bestehend aus Plasmid-DNA und dem K16-VprN-Peptid hergestellt und ebenso zur Transfektion von HCT116-Zellen getestet.
In beiden Anwendungen wurde als Vergleich zum Kl6-VprN-Peptid das K16-mVprN-Peptid verwendet Das Ergebnis zeigt eine deutliche Erhöhung der Genexpression in beiden Anwendungn (vgL Abbildungen 9 und 10).
Diese Sequenz-spezifische Verbesserung der Transfektionseffizienz wurde in besonderem Maße in der Anwendung des Peptides ohne zusätzliche Verwendung des Lipids erzielt (vgl. Abbildung 10). Das K16-VprN-Peptid bewickte eine 10-fache Erhöhung der maximalen Genexpression im Vergleich zum K16-mVprN-Peptid. Ausserdem wurd für das K16-VprN-Peptid für eine maximale Genexpression gleichzeitig eine 12-fach geringere Menge an Peptid benötigt im Vergleich zum K16-mVprN-Peptid.
Um auszuschließen, daß die erzielte Verbessenmg der Transfektionseffizienz auf eine verbesserte Bildung von DNA-Komplexen beruht, wurden die scheinbare Größe (vgl. Abbildung 11) und die relative Partikel-Anzahl (vgl. Abbildung 12) mittels Photonenkorrelationsspektroskopie (PCS) untersucht. Wie in Abbildung 11 zu sehen ist, unterscheiden sich die Größen der DNA-Komplexe mit dem K16-VprN-Peptid nur geringfügig im Vergleich zum K16-mVprN-Peptid. Abbildung 12 zeigt, daß mit zunehmendem DNA/Peptid-Ladungsverhältnis die Streulicht-Intensität ansteigt. Der Vergleich der Abbildungen 10 und 12 zeigt jedoch, daß die gemessenen Streulicht-Intensitäten mit der Genexpression nicht korreliert Insbesondere bewirkt das K16-VprN-Peptid bei einem DNA/Peptid-Ladungsverhältnis von 3 eine 2-fache Erhöhung der Streulicht-Intensität und eine mindestens 100-fache Erhöhung der Genexpression. Bei einem Ladungsverhältnis von 9 verdopplet sich die Streulicht-Intensität im Vergleich zu einem Ladungsverhältis von 3. Die Genexpression erhöht sich jedoch dabei um etwa 10-fach.

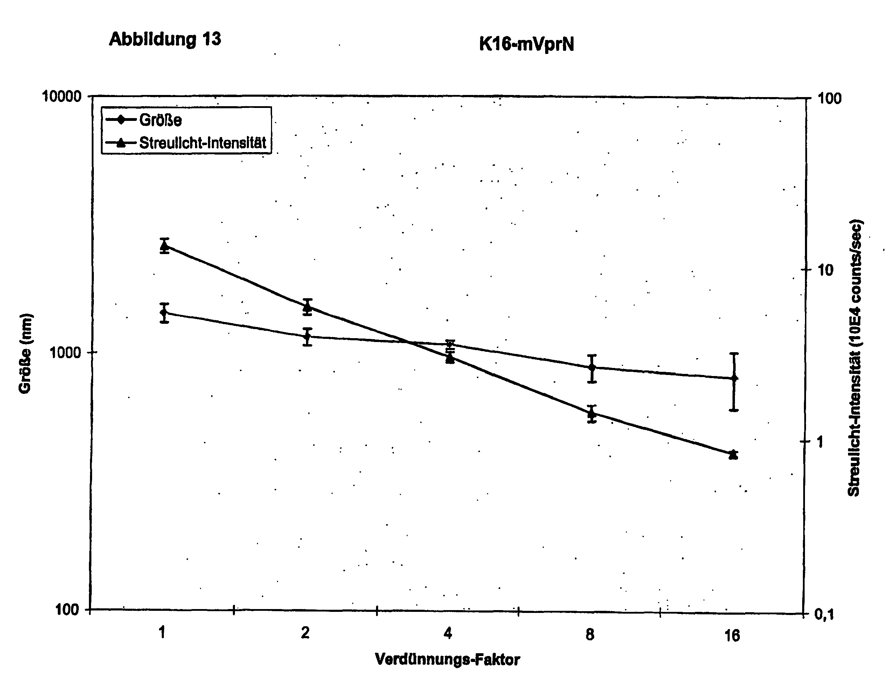

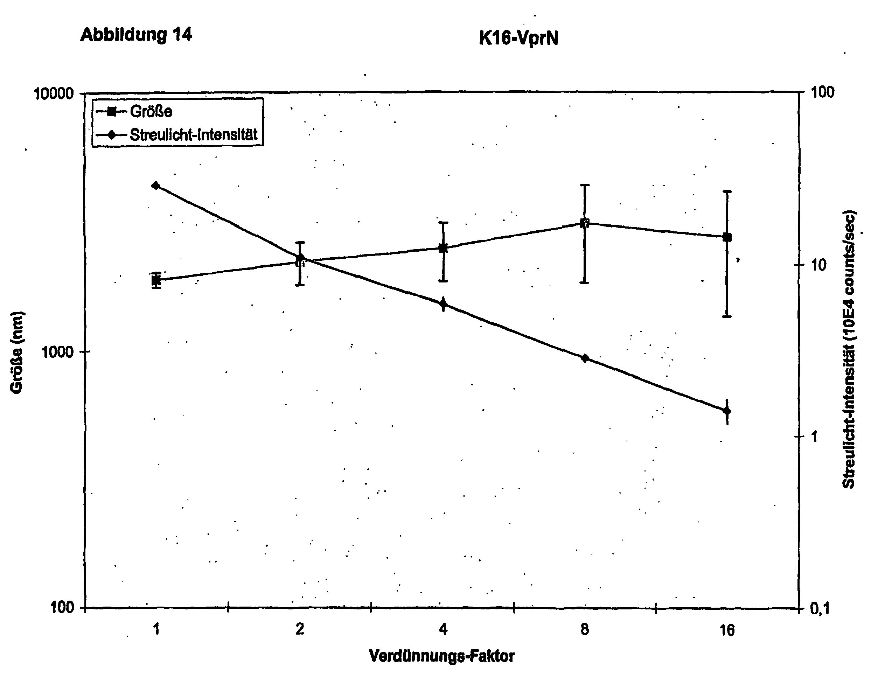

Die Abbildungen 13 und 14 sollen belegen, daß die gemessene Streulicht-Intensität mit der eingesetzten Menge an DNA-Komplexen korreliert. Dazu wurden DNA-Komplexe bestehend aus Plasmid-DNA und den Peptiden K16-VprN und K16-mVprN hergestellt und anschliessend wie abgebildet bis 16-fach verdünnt. Das Ergebnis zeigt, daß die Streulicht-Intensität in dem Maße des Verdünnungsfaktors abnimmt, während die Größe der Partikel etwa gleich bleibt Die Streulicht-Intensität ist also ein Maß für die relative Menge an eingesetzten DNA-Komplexen.

Diese Ergebnisse weisen darauf hin, daß die Sequenz-spezifische Erhöhung der Genexpression durch das K16-VprN Peptid nicht auf eine Erhöhung der Partikelgröße oder der Partikelmenge zurückzuführen ist Folglich ist zu erwarten, daß vielmehr ein zellulärer Mechanismus, beruhend auf die Erkennung der VprN-Sequenz durch spezifische Faktoren während des Transfektionsvorgangs eintritt. Es ist also anzunehmen, daß die K16-VprN/Plasmid DNA-Komplexe von der Kernimport-Maschinerie der Zelle als Importsubstrat erkannt werden, verstärkt in den Kern transportiert werden und dadurch eine Erhöhung der Transfektionseffizienz bewirken.

Zusammengefasst bedeuten diese Resultate in Bezug auf Neuheit, daß:
1. ein DNA-Peptid-Lipid-Komplex konstruiert wurde, dessen Zusammensetzung zu einer Sequenz-spezifischen Erhöhung der Transfektionseffizienz durch eine NLS-Sequenz fuhrt
2. die Zyklisierung einer NLS-Sequenz die Transfektionseffizienz in dem genannten Komplex noch weiterhin erhöht
3. die Verwendung der VprN-Sequenz eine drastische Verbesserung des Transfektionssystems bewirkt.

## Patentansprüche

1. Transfektionsverfahren, **dadurch gekennzeichnet, daß** ein Komplex, bestehend aus
- Plasmid-DNA
- einem synthetischen Peptid und
- einem Lipid
zur Transfektion eingesetzt wird,
wobei
als synthetisches Peptid ein Peptid aus der Gruppe: 
K16-NLS: CKKKKKKKKKKKKKKKKGGGPKKKRKVG
K16-SLN: CKKKKKKKKKKKKKKKKGGGGVKRKKKP
K16-Vpr: KKKKKKKKKKKKKKKKCGGGIGCRHSRIGVTRQRRARNGASRS
K16-VprN KKKKKKKKKKKKKKKKGGGCNEWTLELLEELKNEAVRHF
K16-mVprN KKKKKKKKKKKKKKKKGGGNEATLELLPELKNPAVRHF.
ausgewählt wird oder
als synthetisches Peptid ein zyklisches K16-Peptid, vorzugsweise das K16-CYC, der Peptid-Sequenz: 
K16-CYC: - VKLKVYPLKKKRKP -
KKKKKKKKKKKKKKKKC
verwendet wird.

2. Transfektionsverfahren nach Anspruch 1, **dadurch gekennzeichnet, daß** als Lipid Lipofektin verwendet wird.

3. Transfektionsverfahren nach Anspruch 1 und 2, **dadurch gekennzeichnet, daß** das Ladungsverhältnis Peptid : Plasmid-DNA 0,7 bis 1,0 beträgt.

4. Transfektionsverfahren nach Anspruch 1-3, **dadurch gekennzeichnet, daß** das Mengenverhältnis Plasmid-DNA : Lipid 2 : 1 bis 1 : 1 beträgt.

5. Zyklisches Peptid K16-CYC.

K16-VprN KKKKKKKKKKKKKKKKGGGCNEWTLELLEELKNEAVRHF.

K16-mVprN KKKKKKKKKKKKKKKKGGGNEATLELLPELKNPAVRHF.

8. Verwendung von Peptiden gemäß Anspruch 5 bis 7 in Kombination mit Plasmid-DNA zur Transfektion.

## Claims

1. A transfection method, **characterized in that** a complex consisting of
- plasmid DNA,
- a synthetic peptide, and
- a lipid
is employed for transfection,
a peptide from the group of: 
K16-NLS : CKKKKKKKKKKKKKKKKGGGPKKKRKVG,
K16-SLN : CKKKKKKKKKKKKKKKKGGGGVKRKKKP,
K16-Vpr : KKKKKKKKKKKKKKKKCGGGIGCRHSRIGVTRQRRARNGASRS,
K16-VprN : KKKKKKKKKKKKKKKKGGGCNEWTLELLEELKNEAVRHF,
K16-mVprN : KKKKKKKKKKKKKKKKGGGNEATLELLPELKNPAVRHF
being selected as synthetic peptide, or
a cyclic K16 peptide, preferably K-16 CYC having the peptide sequence 
K16-CYC : - VKLKVYPLKKKRKP –
KKKKKKKKKKKKKKKKC.
being used as synthetic peptide.

2. The transfection method according to claim 1, **characterized in that** lipofectin is used as lipid.

3. The transfection method according to claims 1 and 2, **characterized in that** the charge ratio of peptide/plasmid DNA is from 0.7 to 1.0.

4. The transfection method according to claims 1 to 3, **characterized in that** the quantitative ratio of plasmid DNA/lipid is from 2:1 to 1:1.

5. Cyclic peptide K16-CYC.

K16-VprN KKKKKKKKKKKKKKKKGGGCNEWTLELLEELKNEAVRHF.

K16-mVprN KKKKKKKKKKKKKKKKGGGNEATLELLPELKNPAVRHF.

8. Use of peptides according to claims 5 to 7 in combination with plasmid DNA for transfection.

## Revendications

1. Procédé de transfection, **caractérisé en ce qu'**on utilise pour la transfection un complexe composé
- d'ADN plasmidique,
- d'un peptide synthétique et
- d'un lipide,
moyennant quoi on sélectionne, en tant que peptide synthétique, un peptide appartenant au groupe suivant : 
K16-NLS : CKKKKKKKKKKKKKKKKGGGPKKKRKVG,
K16-SLN : CKKKKKKKKKKKKKKKKGGGGVKRKKKP,
K16-Vpr : KKKKKKKKKKKKKKKKCGGGIGCRHSRIGVTRQRRARNGASRS,
K16-VprN : KKKKKKKKKKKKKKKKGGGCNEWTLELLEELKNEAVRHF,
K16-mVprN : KKKKKKKKKKKKKKKKGGGNEATLELLPELKNPAVRHF,
ou on utilise, en tant que peptide synthétique, un peptide K-16 cyclique, de préférence le K16-CYC ayant pour séquence peptidique :

K16-CYC : - VKLKVYPLKKKRKP –
KKKKKKKKKKKKKKKKC.

2. Procédé de transfection selon la revendication 1, **caractérisé en ce qu'**en tant que lipide, on utilise de la lipofectine.

3. Procédé de transfection selon les revendications 1 et 2, **caractérisé en ce que** le rapport de charge du peptide à l'ADN plasmidique est compris entre 0,7 et 1.

4. Procédé de transfection selon l'une des revendications 1 à 3, **caractérisé en ce que** le rapport quantitatif de l'ADN plasmidique au lipide est compris entre 2:1 et 1:1.

5. Peptide cyclique K16-CYC.

K16-VprN : KKKKKKKKKKKKKKKKGGGCNEWTLELLEELKNEAVRHF.

K16-mVprN : KKKKKKKKKKKKKKKKGGGNEATLELLPELKNPAVRHF.

8. Utilisation de peptides selon l'une des revendications 5 à 7 en association avec de l'ADN plasmidique pour réaliser une transfection.
